# EUROPEAN PATENT APPLICATION

(11) **EP 3 488 705 A1**
(43) Date of publication of application: **29.05.2019**
(21) Application number: 17203360.7
(22) Date of filing: 23.11.2017
(51) Int. Cl.: A23K 20/137, A23K 20/10, A23K 20/168, A23K 20/195, A23K 50/10, A23K 50/75, A23K 50/30

(54) **SOLUBILIZED GROWTH PROMOTING PRODUCT TO BE APPLIED ORALLY TO ANIMALS AND METHOD FOR THE RESPECTIVE FEED PREPARATION**

(71) Applicant: Saviela AG, 6340 Baar (CH)
(72) Inventor: Fehr, Markus, 8031 Zürich (CH)
(74) Representative: Felber, Josef

(57) **Abstract**

The preparing of a growth promoting product, e.g. based on zilpateriol HCI or other growth promoting agents for feeding animals and supporting their weight gain is done by mixing the particular growth promoting agent with e.g. 15 Vol-% propylenglycol in order to obtain a solubilized substance. Various supplemental agents may be added, such as preservatives, inhibotors of bacteria growth, steriods etc. The obtained liquid product is finally diluted with water, ready to be dispersed and mixed with solid feed. Preferably, the solubilized growth promoting product is being sprayed onto the solid feed in a feed mixer in order to homogeneously mixing the product with the feed. The handling of this liquid growth promoting product is easier, safer for the staff working with the product and enhances the homgeneous dispersion in the feed and the handling of the feed is more economic compared to the use of solid agents.

## Description

Growth promotings are natural or synthetically produced substances which enhance the growth and in particular the muscle growth of animals such as cattle, sheep, pigs, poultry and other domestic animals. For example Zilpaterol is a known adrenergic ß-2 agonist corresponding in structure to this formula: 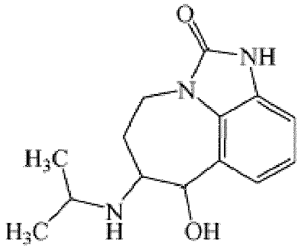 The IUPAC name for zilpaterol is 4,5,6,7-tetrahydro-7-hydroxy-6-(isopropylamino) imidazo [4,5,1-jk]-[1]benzazepin-2(1H)-one. The Chemical Abstracts name for zilpaterol is 4,5,6,7-tetrahydro-7-hydroxy-6-[(1-methyl-ethyl) amino]-imidazo [4,5,1-jk] [1]benzazepin2(1H)-one. Zilpaterol hydrochloride is e.g. sold by Merck Animal Health, under the trademark 10 ZILMAX®. It is approved in the United States for increased rate of weight gain, improved feed efficiency, and increased carcass leanness in cattle fed in confinement for slaughter during the last 20 to 40 days on feed. The approved inclusion rate of zilpaterol hydrochloride is 6.8 grams/ton (7.5 ppm) in feed (see Freedom of Information Summary for NADA 141-258, approved August 10, 2006). This rate, however, varies depending on the feed intake of the animals. At least there it is generally recommended to use 0.15 mg/kg BM and this is being done in practise. The 0.15 mg/kg BM are resulting in the recommended 60-90 mg/head of cattle.

In U.S. Patent 4,585,770, Fréhet et al. discuss compounds, such as zilpaterol, encompassed by a genus characterized as 6-amino-7-hydroxy-4,5,6,7-tetrahydro-imidazo[4,5,1-jk][1]-benzazepin-2-(1 H)-one derivatives and acid addition salts thereof.

In U.S. Patent 4,900,735, Grandadam discusses a zootechnical composition comprising zilpaterol and acid addition salts thereof. Grandadam states that such a composition may be used in general to increase the weight of cattle, pigs, sheep, and poultry.

In U.S. Patents 5,731,028 and 5,847,124, Chevremont et al. discuss crystallized anhydrous zilpaterol hydrochloride, and particularly crystallized anhydrous zilpaterol hydrochloride wherein less than 5% of the crystals have a size of less than 15 µm, and at least 95% of the crystals have a size of less than 250 µm. According to Chevremont et al., such crystals may be incorporated into animal feed to increase body weight and meat quality. Chevremont et al. provide methods for making such crystals, and discuss using the crystals to make animal premixes in which the crystals are secured to a corn cob support having a greater particle size.

WO2013/171330 discloses a method of enhancing the performance of broiler chickens using zilpaterol containing feed.

WO2014/140237 discloses a method of improving the efficiency of beef production from bovine animals which comprises feeding zilpaterol to the animals.

EP 0 197 188 describes a method of preparing non dusty blend of meals or flours with additives for use in fodder manufacturing and or for livestock feeding. Specifically, the method involves the blending of the meal or flour with a nonionic physiologically compatible surfactant such as esters of propylene glycol. The active ingredient exemplified is Carbadox. Zilpaterol is not disclosed.

Ractopamine hydrochloride is sold by Elanco Animal Health under the trademark Optaflexx®. It is approved in the United States for increasing the rate of weight gain, improving feed efficiency in cattle fed in confinement for slaughter during the last 28 to 42 days on feed. The approved inclusion rate of ractopamine hydrochloride is 10 to 30 ppm (see Freedom of Infonnation Summary for NADA 141-221, approved June 13, 2003).

A preferred animal feed premix is comprising zilpaterol or a salt thereof in the range of 0.5 to 2.0 %, further propylene glycol in range of 5-10%; and a solid carrier. In another preferred embodiment, the animal feed premix further comprises water in the range of 1-4%. In another embodiment, the zilpaterol is zilpaterol HCI. Propylen glycol has a conservation effect and is also an inhibitor for the growth of mushrooms and/or bacteria.

In WO 2017/013167 which might be the closest state of the art, a composition of feed is disclosed as follows:
- 0.5-2.0% zilpaterol HCI
- 5-10% propylen glycol
- 1-4% water
- QS milled corncob
This product is a dry product which will then be added to the feed. A liquid Zilpaterol HCI solution is combined with a solid carrier substance and this dry and solid growth promoting product is sold and distributed. The buyer adds it by mixing this dry and solid product into the feed.

In yet another embodiment, the solid carrier is selected from the group consisting of corn cobs, rice hulls, sugar, maltodextrins, tale, cellulose or inixtures thereof, or corn cobs.

This process of feed preparation has the disadvantages of being expensive because of the added safety needed to handle solid product with zilpaterol HCI and due to the dust that is be generated. Typically, a worker takes a plastic bag with e.g. some or several 100 grams of the solid growth promoting product and pours it by hand into a small mixer to prepare a mixed amount of e.g. 20-30kg of corns. He then pours the obtained mixture into the large feed mixer which may contain approx. 10 to 15 tons of feed. This process may be hazardous to the workers. After all the growth promoting product contains an adrenergic ß-2 agonist which may enter the worker's body although workers wear protection glasses and dust masks. But if they e.g. have an open wound, a tiny amount, a powder corn of the product may enter the blood stream which might be hazardous. The usual process for preparing the feed is therefore not free of dangers to the workers. Furthermore, the preparation process also takes time to complete. An additional disadvantage of existing formulations is that it does not mix perfectly homogenious into the feed at concentrations of or below 4ppm. The current manufacturing process, while effective for the current products, which have a zilpaterol concentration of 4.8% as a premix and are being used to produce feed with a zilpaterol concentration of 6.8 g/ton or 7.5 ppm, is not effective in producing the premix product. It is hard to produce a feed with uniform distribution of zilpaterol in a feed at concentrations of 4 ppm or below. The relative standard deviation (RSD) of feed prepared from a zilpaterol formulation with zilpaterol concentration of 4ppm and manufactured as described is 71.9%. Typically, a feed should have an RSD of less than 10%. Furthermore, medicated premixes have a tendency to cake/lump with prolonged storage. One possible solution would be to evaporate the excess moisture through heat and/or pressure. However, this would increase the expenses and time to manufacture the product.

It is therefore the objective of this invention to provide an improved growth promoting product which allows an easier handling, a more homogeneous dispersion in the feed and a better and more economic way of application, and a more reliable oral administration to animals. Furthermore, the object of this invention is to provide a method for the preparation of such growth promoting product and its use.

This objective is achieved by a growth promoting product for domestic animals, administrable orally with solid feed or in drinking water, characterized in that the growth promoting is solubilized by a solubilization agent and therefore transformed into a liquid substance which is homogeneously and finely mixable into solid feed or homogeneously and finely mixable into drinking water of the animals to be fed. The method for the preparation of such growth promoting product is characterized in that the growth promoting substance or one of its salts is dissolved in a solubilizing agent in order to obtain a liquid product to be mixed and dispersed in the animal feed or drinking water to be fed.

This method and product will be further described in the following, by way of example, in relating to the figures.
They show:
- Figure 1:: An apparatus for dosing the liquid growth promoting product to a water flow;

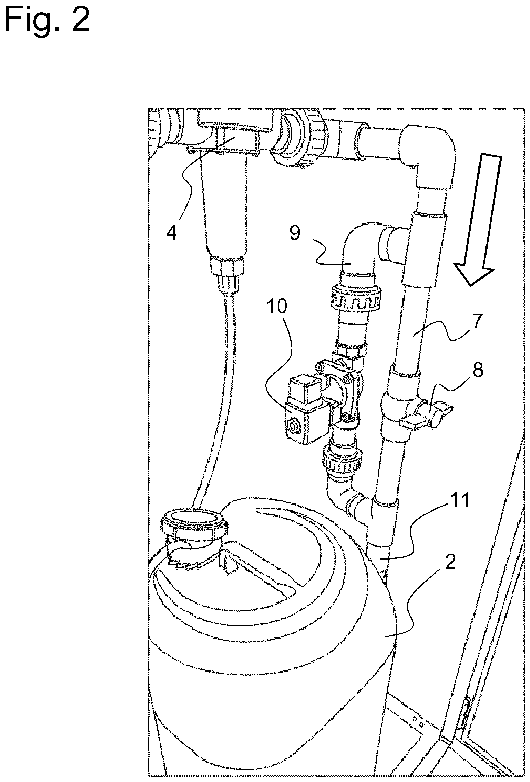

- Figure 2:: The bypass of the apparatus with a solenoid for its control.

This growth promoting product for domestic animals can be administered orally with solid feed or in drinking water. For this purpose, a growth promoting substance is solubilized by a solubilization agent and therefore transformed into a liquid substance or product, which is homogeneously and finely mixable into solid feed or homogeneously and finely mixable into drinking water of the animals to be fed. In addition, such product may e.g. contain one or several of the following solubilizing agents:
- Propylene glycol
- Pyrrolidone
- N-Methylpyrrolidone (NMP)
- DMSO
- Alcohols
Propylene glycol is a preferred solvent, but a number of other non-aqueous solvents or a combination of non-aqueous solvent and water solvent may be used with propylene glycol comprising the major liquid constituent.

The growth promoting product may further contain one or several preservative(s) and/or antioxidant(s), which is/are being mixed into the growth promoting product. Suitable examples of preservatives are the following:
- E216, that is Propylparaben = Propyl 4-hydroxybenzoate
- E218, that is Methylparaben = Natriumpropyl-p-hydroxybenzoat
- E223, that is sodium metabisulfite
- E270, that is lactic acid as an acidifier
- E280, that is propionic acid as antimicrobial and antioxidant
- E297, that is fumaric acid
- Benzyl alcohol
- Ascorbic acid

The growth promoting product, when based on Beta₂-agonists (β₂-agonists), may contain one ore more of these substances:
- Clenbuterol or one its salts
- Isoproterenol or one of its salts
- Cimaterol or one of its salts
- Ractopamine or one of its salts
- Salbutamol or one if its salts
- Zilpaterol or one of its salts
- Zilpaterol HCI

In case the growth promoting product should have an antimicrobial effect, the following substances are suitable:
- Chlortetracycline
- Zinc bacitracin
- Flavophospholipol
- Monensin
- Lasalocid
- Tylosin
- Salinomycin
- Virginiamycin

In case the growth promoting product contains a steroid as growth promoting substance, it is suitable when it also contains one or a several of the following substances:
- Oestrogenic agents
- Progestational agents
- Androgenic agents

The growth promoting product may on top contain one or several of the following substances:
- a growth hormone
- a set of growth hormones
- Somatotropin

According to the particular method for the preparation of such a growth promoting product for domestic animals, a the growth promoting substance or one of its salts is being mixed into a solubilizing agent in order to obtain a liquid to be mixed and dispersed in the solid animal feed or drinking water to be fed. E.g. zilpaterol or one of its salts can be dissolved in a solubilizing agent without the addition of water and then the obtained product can be mixed and dispersed into the solid animal feed or drinking water. Particularly, zilpaterol or one of its salts are being mixed with a solubilizing agent and water, whereby the percentage of the solubilizing agent in said mixture is in the range of 1% to 99%.

The obtained growth promoting products are advantageously administered by spraying the product into solid animal feed and homogeneously mixing it with said feed, e.g. in a feed mixer commonly used to make a TMR (total mixed ration) for animals kept in intensive production systems. As an alternative, the oral administering of the growth promoting product can be achieved by adding the liquid product to the drinking water and mixing it with said water which is then provided as drinking water to the animals. It is also possible to mix the liquid product with a variable amount of water before mixing the obtained mixture into the feed or water to increase the homogeneity of the final feed or water. Eventually, the liquid growth promoting product can be dosed by using a dosing system to mix the product with water or with a premix before mixing the final product into solid feed or water.

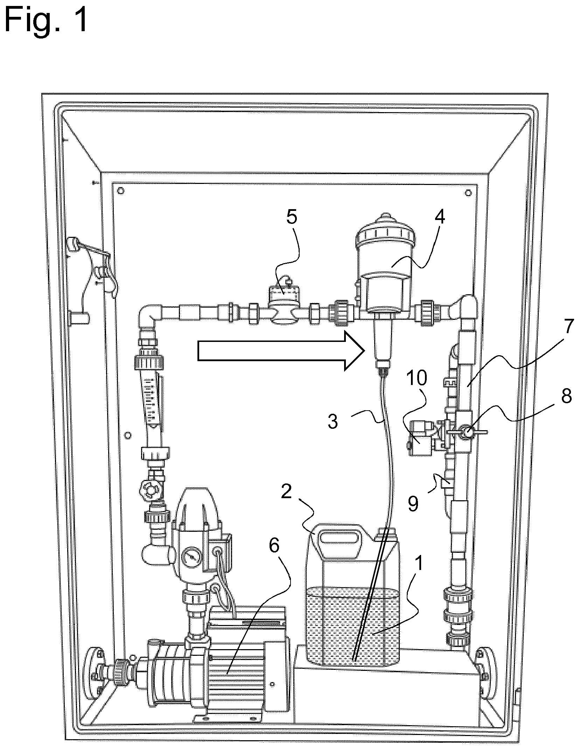

The mixing of the liquid growth promoting product with water before adding it to the solid feed may be performed by a particular apparatus which is shown in figure 1. The liquid growth promoting product 1 is contained in the canister 2. A flexible tube 3 extends from the bottom of this canister 2 to a dosing apparatus 4 through which a stream of water is being pumped, in figure 1 in clock-wise direction. The water comes from a waterline with adequate pressure or is being pumped by an electrical pump 6, then flows through the water meter 5 into the dosing apparatus 4, here as an example of the brand Dosatron of Dosatron International. Inc., Rue Pascal, F-33370 Tresses, France. Dosatron is a solution for accurately dose a liquid or soluble concentrate into water, acid for example, and prepare an 'aqueous solution', or even dose and mix other liquids. This dosing apparatus 4 by the force of the water stream sucks a finely and accurately dosable amount of the liquid growth promoting product 1 into the water stream. The mixture then passes down the pipe 9 which is equipped with a solenoid powered valve 10 which can close or open said bypass electrically to automatically regulate the amount of water-growth promoting agent mixture that is added to the feed. The solenoid 10 can be bypassed through pipe 7, if the manual bypass switch 8 is opened. By this arrangement, a dosing can be performed even if there is a breakdown of the electric power supply, namely without the solenoid. Instead of mixing by using an advanced dosing system, e.g. the Dosatron, the preparation may also be mixed by other dosing technologies or by hand or in a water tank before the mixture of the growth promoting agent and water is added to the feed. The reason to mix it first with water is to improve the spread and the homogeneity in the feed, since it would be difficult to homogeneously add 50-150 ml of the product to a ton of feed. But if the volume will be increased to about 5-30 litres per ton of feed, it can be added and mixed very well and equal. The entire apparatus can be controlled by a programmable electronic control unit (not shown). The output of the pump 6, the dosing of the dosing apparatus 4 and the solenoid valve 10 can be controlled by said control unit which also receives the data of the water meter 5. This allows it to produce a uniform and highly homgeneous mixture of the liquid growth promoting product with water. The mixture can then be sprayed onto the feed in a feed mixer.

In figure 4 the bypass pipe 9 beside the regular pipe 7 with its solenoid valve 10 is shown. When the valve 8 in the direct line 7 is closed, the solenoid valve 10 regulates the flow through in the bypass pipe 9 and therefore the discharge of mixture can be regulated by the control unit. The discharged mixture is led by the endpipe 11 to a feed mixer (not shown) in which the solid feed is contained. In said feed mixer the fully prepared solution is being sprinkled through one or several oulets directly onto the feed in the feed mixer and ensures a homogeneous dispersion. A spraying via nozzles is prevented in order to prevent the liquid solution to evaporize into the air. Rather, the outlets only produce dropping liquid flow into the feed.

As mentioned above, the watery solution of the growth promoting product can then directly be added in this way as liquid solution to solid animal feed, depending on the desired amount to be fed. E.g. milled corn cobs are a preferred carrier. Other dry carriers can be used including rice hulls and other grain materials with good absorptive properties. Other carries like sugars/maltodextrins, tale, celluloses etc. can be used alternatively, as long as they have similar absorptive properties. It is also possible to use a combination of such carriers. The choice of the carriers is determined by its adsorptive properties and the physical characteristics to allow homogeneous mixing with grain or other conventional feeds in order to form a medicated feed.

As a none limiting example, some 0.15 mg of pure zilpateriol HCI are to be fed per kg of body weight of a particular animal per day. For sheep this means some 70 ml of the liquid growth promoting liquid per ton of feed and for beef some 125 ml per ton of feed. These amounts can though vary depending on the daily feed intake, the anmial breed, the species (e.g. cattle, sheep, pigs or poultry) and other circumstances. It is a challenge to prepare the feed in such way that for an animal weighing 500 kg, e.g. only 75 mg of Zilpateriol HCI is reliably contained in the 13 kg = 13'000'000 mg of solid feed which the annimal will eat per day. Hence only 0.00057% of the feed weight is the actually zilpateriol HCI in this example.

Depending on what kind of feed is being used and on the dry substance of such feed, a respective amount of this liquid growth promoting product can be mixed into the feed. There are conventional mixers which can be used for this mixing process. These mixers commonly have one or several rotating, horizontal or vertical augers with mixing knives and large amounts of feed can be mixed in such mixers, e.g. single pieces of 500kg hey bales or other solid feed such as molasse flour, crops, grass etc. can be added to the mixer together with other ingredients. Depending on a particular need, vitamin mixtures, sugars, maltodextrins, tale, celluloses may be added and eventually, the liquid product containing the e.g. zilpateriol HCI or other growth promoting agents is being sprinkled into the operating feed mixer, thereby ensuring a homogeneous mixing and dispersion of the product into the entirety of the contained feed. Different models of commonly used feed mixers can mix feed ranging from a few hundred kilograms to many tons per load..

The need of dry substance of feed does vary from animal species to species. Feedlot cattle typically eat approx. 2.6 % of their body weight in dry feed substance per day, while feedlot sheep eat approx. 4.5 %. Bascially, the smaller the animal species is, the higher said percentage will be.

Smaller commercial feedlots can stock 1000 to 1500 cattle and run three cycles per year, i.e. slaughtering 3000 to 4500 animals per year. There are also some farms which stock several 10'000 cattle at any given time. Usually some 100 to 150 cattle are in one single paddock. The feeding troughs can be many 100 meters long. It is clear that they need to be filled by a particular feeding machine which will be driven along the feeding trough. Now, once the liquid growth promoting product is throughly and homogeneously mixed into the solid feed, the TMR (total mixed ration) can be discharged in a continuous stream from the mixing wagon or from a delivery vehicle into the feeding troughs while driving the feeding machine along the trough. This described preparation of the feed does ensure a homogeneous dispersion of the actually very small amount of growth promoting product among the large amount of feed.

Other additives may be included in either the growth promoting product's solution phase or solid carrier including colorants, preservatives, buffers, glidants, stabilizers, crystallization inhibitors. An example of a crystallization inhibitor is polyvinylpyrrolidone (PVP). The medicated feed that is produced with the feed premix according to the current invention is especially useful for improving the growth of meat producing animals, such as e.g. cattle, especially beef cattle in a feedlot, sheep of different breeds in a feedlot, pigs and poultry, especially broilers.

By using the presented method for the preparation and ultimate oral adminstration of this product, no physical contact of the product with the people who prepare and add it is possible. The liquid product is being prepared and added to the feed in a definite, always constant ratio and fully automatical manner. This enhances the saftey of the people working with the product and facilitates its administration. And on top the animals get their exact ration since the product is uniformly dispersed in the feed.

## Claims

1. Growth promoting product for domestic animals, administrable orally with solid feed or in drinking water, **characterized in that** the growth promoting product is solubilized by a solubilization agent and therefore transformed into a liquid substance which is homogeneously and finely mixable into solid feed or homogeneously and finely mixable into drinking water of the animals to be fed.

2. Growth promoting product for domestic animals according to claim 1, **characterized in that** the product contains e.g. one or several of the following solubilizing agents:
• Propylene glycol
• Pyrrolidone
• N-Methylpyrrolidone (NMP)
• DMSO
• Alcohols

3. Growth promoting product for domestic animals according to one of the preceding claims, wherein the growth promoting product contains at least one preservative.

4. Growth promoting product for domestic animals according one of the preceding claims, wherein the growth promoting product contains a mixed in preservative before mixing the product into solid feed or drinking water, the preservative being e.g. one or more of the following selection:
• E216, that is Propylparaben = Propyl 4-hydroxybenzoate
• E218, that is Methylparaben = Natriumpropyl-p-hydroxybenzoat
• E223, that is sodium metabisulfite
• E280, that is propionic acid, as antimicrobial and antioxidant
• E297, that is fumaric acid
• Benzyl alcohol
• Ascorbic acid

5. Growth promoting product for domestic animals according to one of the preceding claims, **characterized in that** the product contains one or several substances which are Beta₂-agonists (β₂-agonists), e.g. of the following selection:
• Clenbuterol or one its salts
• Isoproterenol or one of its salts
• Cimaterol or one of its salts
• Ractopamine or one of its salts
• Salbutamol or one if its salts
• Zilpaterol or one of its salts
• Zilpaterol HCI

6. Growth promoting product for domestic animals according to one of the preceding claims, **characterized in that** the product contains an antimicrobial growth promoting product and contains one or a several substances, e.g. of the following selection:
• Chlortetracycline
• ZincBacitracin
• Flavophospholipol
• Monensin
• Lasalocid
• Tylosin
• Salinomycin
• Virginiamycin

7. Growth promoting product for domestic animals according to one of the preceding claims, **characterized in that** the product contains a steroid as growth promoting product and contains e.g. one or several substances of the following selection:
• Oestrogenic agents
• Progestational agents
• Androgenic agents

8. Growth promoting product for domestic animals according to one of the preceding claims, **characterized in that** the product contains one or several of the following substances:
• a growth hormone
• a set of growth hormones
• Somatotropin

9. Method for the preparation of a growth promoting product for domestic animals according to one of the preceding claims, **characterized in that** a solubilizing agent is used to dissolve the growth promoting substance or one of its salts in said agent in order to obtain a liquid substance to be mixed and dispersed in the animal feed or drinking water to be fed.

10. Method for the preparation of the growth promoting product for domestic animals according to one of the preceding claims, **characterized in that** the growth promoting product is prepared by using a dosing system, by adding e.g. 0.8% of said liquid growth promoting product per 1 litre of water in a flow through apparatus containing a pump (6), a water meter (5) and a dosing apparatus (4) which sucks the liquid substance (1) finely adjustable out of a canister (2) by the throughflow of the pumped water, and flowing through a bypass (9) with a solenoid valve (10), whereby the pump (6), the dosing apparatus (4) as well as the solenoid valve (10) of the bypass (9) are controllable by a programmable electronic control unit.

11. Method for the preparation of a growth promoting product for domestic animals according to one of the claims 1 to 8, **characterized by** dissolving zilpaterol or one of its salts in a solubilizing agent without the addition of water and then mixing and dispersing the product into the animal feed or drinking water.

12. Method for the preparation of a growth promoting product for domestic animals according to one of the claims 1 to 8, **characterized by** dissolving zilpaterol or one of its salts into a mixture of a solubilizing agent and water, whereby the percentage of solubilizing agent in the mixture is in the range of 1% to 99%.

13. Method for the oral administration of the growth promoting product for domestic animals according to one of the claims 1 to 8, **characterized in that** the growth promoting product is administered by spraying it into a feed mixer containing the solid feed and dispersing the product homogeneously within the animal feed, by spraying it onto the solid feed in a ratio of 0.15 mg per kg body weight of the animals to be fed.

14. Method for the oral administration of the growth promoting product for domestic animals according to one of the claims 1 to 8, **characterized in that** the growth promoting product is administered by spraying it into a feed mixer containing the solid feed and dispersing it homogeneously within the animal feed, by adding it to the solid feed at a concentration that will result in a dosage of 0.15 mg per kg body weight TMR (total mixed ration) for feedlot cattle which is typically about 125 g of zilpaterol per ton of dry matter of a TMR (total mixed ration).

15. Method for the oral administration of the growth promoting product for domestic animals according to one of the claims 1 to 8, **characterized in that** the growth promoting product is administered by spraying it into a feedmixer containing the solid feed and dispersing it homogeneously within the animal feed, by adding it to the solid feed in at a concentration that will result in a dosage of 0.15 mg per kg body weight of the animals to be fed for feedlot sheep which is typically about 75 g of zilpaterol per ton of dry matter of a TMR.
